(19) **Europäisches Patentamt**

**European Patent Office**

**Office européen des brevets**

(11) Numéro de publication: **0 511 970 B1**

(12) **FASCICULE DE BREVET EUROPEEN**

(45) Date de publication de fascicule du brevet: **21.06.95**

(51) Int. Cl.6: **A23J 3/34**, C12P 21/06, C12S 3/14, C07K 2/00, //A61K31/00,A23J3/04,A23J3/14

(21) Numéro de dépôt: **91901506.5**

(22) Date de dépôt: **11.01.91**

(86) Numéro de dépôt internationale : **PCT/BE91/00001**

(87) Numéro de publication internationale : **WO 91/10369 (25.07.91 91/17)**

(54) **PROCEDE POUR PREPARER UN HYDROLYSAT ENZYMATIQUE.**

(30) Priorité: **12.01.90 BE 9000044**

(43) Date de publication de la demande:
**11.11.92 Bulletin 92/46**

(45) Mention de la délivrance du brevet:
**21.06.95 Bulletin 95/25**

(84) Etats contractants désignés:
**AT BE CH DE DK ES FR GB GR IT LI LU NL SE**

(56) Documents cités:
**EP-A- 0 044 032      EP-A- 0 065 663
EP-A- 0 148 072      EP-A- 0 274 939
EP-A- 0 274 939      EP-A- 0 274 946
FR-A- 2 367 773      US-A- 3 857 966**

(73) Titulaire: **TESSENDERLO CHEMIE N.V.
Stationsstraat
B-3980 Tessenderlo (BE)**

(72) Inventeur: **LOOSEN, Pierre, C.
Russelenberg 34
B-3980 Tessenderlo (BE)**
Inventeur: **BRESSOLLIER, Philippe, R.
16, rue Camille-Jullian
F-87000 Limoges (FR)**
Inventeur: **JULIEN, Raymond, A.
65, avenue Baudin
F-87000 Limoges (FR)**
Inventeur: **PEJOAN, Claude, H.
Chabanne
F-87240 S.-Sylvestre (FR)**
Inventeur: **VERNEUIL, Bernard, G.
Les Levades
F-87430 Verneuil-sur-Vienne (FR)**

(74) Mandataire: **Vanderperre, Robert et al
Bureau Vander Haeghen S.A.
Rue Colonel Bourg 108 A
B-1040 Bruxelles (BE)**

Rank Xerox (UK) Business Services
(3.10/3.09/3.3.3)

EP 0 511 970 B1

## Description

L'invention est relative à un procédé pour préparer un hydrolysat enzymatique contenant des di et tripeptides, à partir d'un mélange de protéines à l'aide d'enzymes protéolytiques.

La présente invention a encore pour objet un hydrolysat riche en di et tripeptide.

L'importance des hydrolysats riches en di et tripeptides et à faible teneur en acides aminés libres est largement reconnue par de nombreuses études scientifiques.

L'importance physiologique de l'adsorption intestinale des di et tripeptides a été découverte depuis environ treize ans. Ainsi, l'existence de systèmes de transport intestinal des petits peptides, différents de ceux des acides aminés libres et la plus grande efficacité de l'absorption lorsque l'apport alimentaire des protéines est réalisé à partir de mélanges enrichis en petits peptides ont été démontrées par SLEISENGER et al dans un article intitulé "Evidence for a single common carrier for uptake of a dipeptide and tripeptide by hamster jejunum in vitro" publié dans la revue "Gastroenterology", volume 71, pages 76 à 81 (1976).

Des compositions riches en di et tripeptides présentent un réel intérêt en diététique non seulement pour l'alimentation des jeunes enfants, des convalescents et des anémiques, mais aussi pour celle des malades dont l'absorption intestinale est altérée, tels que des patients atteints de la maladie de HARTNUP ou de cystinurie.

Tant chez les sujets atteints de la maladie de HARTNUP que chez ceux atteints de cystinurie, les acides aminés sont absorbés normalement par la muqueuse intestinale s'ils sont présentés sous forme dipeptidique.(voir BRINSON, R.R., HANUMANTHU, S.K., and PITTS, W.M. (1989) : "A reappraisal of the peptide based enteral formulas : clinical applications". Nutritional in Clinical Practice 4 : 211 - 217. NAYAB, F. and ASATOOR, A.M. (1970) : "Studies on intestinal absorption of amino acids and a dipeptide in a case of Hartnup disease." Gut (Journal of the British Society for Gastroentology) volume 11, pages 373-379 (1970).

L'invention trouve dès lors, par exemple, une application en alimentation hospitalière, en particulier en nutrition artificielle administrée par voie orale, entérale ou parentérale, plus spécialement par infusion intraveineuse.

La nutrition artificielle s'impose aux patients incapables de s'alimenter par la voie normale en raison d'une incapacité due à un dommage physique causé par un accident, une opération chirurgicale, un traumatisme de l'oesophage ou en raison d'un état physiologique général qui ne permet pas une alimentation normale (coma, brûlures).

D'autres applications possibles sont l'immuno-stimulation ou l'alimentation animale, spécialement la nourriture pour poissons, ou l'utilisation comme stimulateur de croissance.

On connaît diverses méthodes pour préparer des hydrolysats de protéines contenant des di et tripeptides : l'hydrolyse alcaline ou acide, l'hydrolyse enzymatique et la synthèse chimique. Toutefois, toutes ces méthodes connues ne permettent pas de préparer à partir de protéines animales et/ou végétales un hydrolysat contenant au moins 75 % en moles de di et tripeptides.

Les protéines utilisables à l'échelle industrielle pour la fabrication d'un hydrolysat sont à titre d'exemple les suivantes :

- protéines de l'oeuf (ovalbumine);
- les protéines du lait : caséine, lactosérum, lactalbumines, lactoglobulines:
- les protéines du sang d'abattoirs : plasma sanguin, sérum albumine, hémoglobine décolorée;
- les produits de l'industrie de la pêche et de la conserverie de poissons, et
- les protéines d'origine végétale : protéines de soja et de luzerne.

L'hydrolyse alcaline ou l'hydrolyse acide de mélanges de protéines tels que la lactalbumine, l'ovalbumine, le lactoserum et la caséine au moyen d'acides ou de bases fortes est utilisée à haute température pour rompre les liaisons chimiques. Elle conduit à l'obtention de mélanges considérablement enrichis en acides aminés libres et souvent très fortement contaminés en sels ($Cl^-$, $Na^+$) qui sont difficiles à éliminer.

Les méthodes chimiques connues sont faciles à mettre en oeuvre et permettent d'obtenir d'importants degrés d'hydrolyse par rupture d'un nombre important de liaisons peptidiques. Elles sont cependant drastiques et entraînent la formation de réactions secondaires indésirables ainsi qu'une diminution de la valeur nutritionnelle des protéines par la dégradation d'acides aminés essentiels.

Ainsi, l'hydrolyse alcaline à haute température (100 à 110°C), engendre souvent la formation de lysinoalanine, un sous-produit très toxique. En outre, il est difficile de contrôler le degré d'hydrolyse du produit fini.

Les enzymes protéolytiques utilisables pour l'hydrolyse enzymatique sont des enzymes d'origine bactérienne, fongique, animale et/ou végétale.

L'action de l'une ou de plusieurs de ces enzymes sur l'une ou plusieurs de ces protéines conduit à des hydrolysats contenant au plus 50 % de di et tripeptides.

Par le document EP-A-0274 946, on connaît un procédé pour fabriquer un hydrolysat enzymatique à haute teneur en di et tripeptides et faible concentration en acides aminés libres, utilisable notamment en nutrition artificielle et en diététique. Dans ce procédé connu, une source protéique classique est soumise à l'action - simultanée ou séquentielle d'un mélange de trois enzymes dans les conditions d'hydrolyse suivantes :

- un pH neutre à alcalin de 7 à 10;
- une température de 20 à 70°C,
- une durée d'hydrolyse égale ou inférieure à 300 minutes.

La haute teneur en di et tripeptides et la faible concentration en acides aminés libres ne sont obtenues qu'après purification de l'hydrolysat enzymatique par un procédé de chromatographie d'échange d'ions tel que décrit dans le document EP-A-0274939 analysé ci-après. Cette étape supplémentaire a l'inconvénient de diminuer le rendement net du procédé en di et tripeptides de 25%.

Le procédé de préparation d'un mélange peptidique riche en di et tripeptides, utilisables en nutrition artificielle et en diététique, décrit dans le document EP-A-0274939 comprend la chromatographie sur une colonne d'une résine échangeuse de cations sous forme acide des di et tripeptides à partir d'un hydrolysat enzymatique de protéines contenant 10 à 50 % en poids de di et tripeptides ainsi que des polypeptides supérieurs aux tripeptides.

Le substrat de l'hydrolyse enzymatique est constitué d'une ou plusieurs protéines animales ou végétales, par exemple protéine de l'oeuf, du lait, du sang d'abattoirs et/ou de soja, de luzerne. L'hydrolysat enzymatique est purifié par ultrafiltration. On dépose ensuite l'hydrolysat purifié sur une colonne de résine échangeuse de cations pour augmenter les proportions en di et tripeptides dans le mélange.

Malgré la mise en oeuvre d'un mélange d'enzymes, l'hydrolysat enzymatique non enrichi sur une colonne de résine échangeuse de cations contient au plus 50% de di et tripeptides, au moins 7% d'acides aminés libres ainsi qu'une teneur trop élevée en ions (voir tableau 1 à la page 8 du document cité).

L'hydrolysat est purifié sur une colonne de résine échangeuse de cations afin d'augmenter les proportions de di et tripeptides dans le mélange et de diminuer la concentration en sels. Cette étape d'enrichissement en di et tripeptides présente les inconvénients de diminuer le rendement en poids mais également d'être lourde et coûteuse.

Le document EP-A-0148072 décrit un procédé de protéolyse de protéines plasmatiques en vue de fournir des hydrolysats protéiques appropriés à l'alimentation humaine et animale grâce à leur saveur et leur goût acceptables. Dans ce procédé, le plasma sanguin servant de substrat à l'hydrolyse est obtenu par centrifugation du sang total recueilli sous anticoagulant à l'abattage. Par centrifugation dans un séparateur on obtient une phase légère jaune, le plasma, et une phase lourde rouge, le cruor.

On effectue au préalable une dénaturation du plasma par traitement thermique des protéines à une température de 70 à 100°C, le pH étant maintenu à une valeur de 5 à 7 pendant moins de 30 minutes, en vue d'augmenter le rendement de la protéolyse.

On effectue ensuite la protéolyse des protéines plasmatiques en ajoutant celles-ci à un milieu réactionnel aqueux contenant de la thermolysine, en présence d'ions calcium à un pH de 5 à 7 et à une température inférieure ou égale à 80°C.

Par l'utilisation d'une gamme de températures comprise entre 50 et 60°C, on évite la prolifération des bactéries mésophiles.

Le choix judicieux des enzymes et des conditions d'hydrolyse conduit à l'obtention de peptides de tailles définies selon les besoins.

L'inconvénient de ce procédé connu réside entre autre dans le faible rendement pondéral en di et tripeptides de la protéolyse. En effet, la filtration de l'hydrolysat sur Biogel P6 met en évidence la présence de peptides avant une masse molaire inférieure à 2000, c'est-à-dire un mélange d'acides aminés libres et de peptides de 2 à 10 acides aminés, la teneur en di et tripeptides restant très faible. Or en nutrition artificielle, les peptides de plus de trois acides aminés ne procurent pas les avantages cinétiques de l'absorption décrite précédemment.

Par le document EP-A-0065663 (MILES) on connaît une méthode pour préparer un hydrolysat enzymatique diététique contenant des di et tripeptides à partir d'un mélange de protéines de petit lait à l'aide d'enzymes fongiques. Cette méthode connue comprend les étapes suivantes :

- sélection d'un substrat formé d'un mélange de protéines utilisables;
- traitement thermique préalable de mise en forme d'un substrat pour augmenter l'accessibilité du mélange de protéines aux enzymes, mis en oeuvre dans une étape ultérieure;
- hydrolyse enzymatique; et

- séparation liquide/solide de l'hydrolysat.

Le traitement thermique est effectué à pH 8 et ne comporte pas d'étape de séparation liquide solide avant hydrolyse.

La méthode ne s'applique qu'aux mélanges et protéines de petit lait et non pas à divers substrats. Elle comprend un traitement préalable mettant en oeuvre les conditions opératoires suivantes : pH = 8; T = 100-110°C; t = 10 minutes et ne permet d'obtenir qu'un rendement d'hydrolyse d'environ 40%, tout au plus 58,2%. L'hydrolysat contient 65% d'acides aminés et di- et tripeptides, 20% de polypeptides ayant 4-9 acides aminés et 16% de polypeptides ayant 10 ou plus d'acides aminés. Le procédé ne comporte pas d'étape d'ultrafiltration.

La présente invention vise à remédier à ces inconvénients. Elle a pour objet un procédé pour préparer un hydrolysat enzymatique riche en di et tripeptides à partir d'un mélange de protéines à l'aide d'enzymes protéolytiques. Le procédé est caractérisé en ce qu'on met le mélange de protéines de départ en forme par traitement thermique dans un intervalle de température compris entre 70 et 100°C à pH 4,5 à 6 pour le plasma sanguin, à ph 4,0 à 5,0 pour le lactosérum, l'isolat de poisson, l'isolat de pois, l'isolat de soja et l'isolat de froment, à pH 6 à 8 pour la caséinate et à pH 6-7 pour les protéines de l'oeuf entre 30 et moins de 45 minutes et on soumet le mélange mis en forme à une hydrolyse enzymatique, à l'aide d'une seule enzyme additionnée en proportion de 1 à 4% en poids par rapport au substrat, de manière à obtenir un hydrolysat contenant 75% en moles de di et tripeptides avec un rendement d'hydrolyse d'au moins 70% en poids d'hydrolysat par rapport au mélange de protéines, on extrait l'hydrolysat par séparation liquide/solide, puis on en élimine, par ultrafiltration, des protéines solubles partiellement hydrolysées ainsi que les peptides ayant une masse moléculaire supérieure à 10000, de manière à réduire la teneur en acides aminés libres à moins de 5% en moles et la teneur en polypeptides supérieurs à 3 acides aminés à moins de 20% en moles, sans altérer le rendement net en di et tripeptides du procédé.

Le procédé suivant l'invention présente, par exemple, l'avantage de permettre une transformation d'au moins 70% en poids de protéines en une fraction peptidique contenant au moins 75% en moles de di et tripeptides.

Le traitement thermique des protéines provoque une modification de la structure tridimensionnelle de celles-ci en agissant sur les liaisons faibles responsables de la conformation native. Il conduit à une plus grande exposition des liens peptidiques constitutifs de la structure primaire et ainsi améliore l'accessibilité du substrat aux enzymes.

Selon une particularité de l'invention, après avoir dissous un substrat ou mélange de protéines dans de l'eau, on soumet la solution ainsi obtenue à un traitement thermique à une température comprise entre 70 et 100°C, avantageusement pendant une période de temps inférieure à 45 minutes à pH initial ou pH acide ou voisin de la neutralité de manière à obtenir une suspension aqueuse protéique qu'on soumet enfin à une hydrolyse en présence d'enzymes protéolytiques. De façon avantageuse, on dissout le substrat ou mélange de protéinies dans de l'eau de manière à obtenir une solution dont la concentration en substrat ou protéines est inférieure à 100 g/l, de préférence d'environ 70 g/l (N x 6,25). Le traitement thermique est réalisé pendant une durée comprise entre 30 et moins de 45 minutes. L'hydrolyse est réalisée à des valeurs de pH comprises entre 6,5 et 9, de préférence 7 et 7,5 et à des températures comprises entre 50 et 60°C pendant 5 à 9 heures.

Le procédé selon l'invention s'applique à diverses sources protéiques telles que :
les protéines du sang d'abattoirs;
les protéines du lait : caséinate, lactosérum, $\alpha$-lactalbumine, $\beta$-lactoglobuline:
les protéines du soja, du pois et/ou froment, les isolats de poisson, les protéines du blanc d'oeuf, etc.

Dans un mode particulier de mise en oeuvre d'un procédé suivant l'invention, on recueille du sang animal en ajoutant moins de 4 g/l d'anti-coagulant et on le soumet à une centrifugation de manière à obtenir un produit utilisable extemporanément ou apte à la conservation par congélation ou séchage par atomisation, puis on soumet le plasma sanguin ainsi obtenu après ajustement à pH acide tel qu'un pH de 4,5 à 5, à un traitement thermique dans un intervalle de températures comprises entre 70 et 80°C pendant une période de temps inférieure à 45 minutes, de préférence comprise entre 30 et 45 minutes, et ensuite à une séparation liquide-solide, par microfiltration tangentielle, centrifugation, filtration sous pression ou dépression (de préférence sur filtre presse), de manière à obtenir un substrat solide thermocoagulé qu'on soumet enfin à l'hydrolyse en présence d'enzymes protéolytiques après remise en suspension.

Pour l'hydrolyse, l'enzyme utilisée est avantageusement une protéase utilisable en milieu alcalin et est, de préférence, une protéase alcaline à serine en particulier une subtilisine de Bacillus licheniformis (Pescalase® - Gist Brocades; Alcalase® - NOVO), une subtilisine de Bacillus subtilis (Orientase 100® - HANKYU. Japon), une subtilisine de Bacillus amylo-quefaciens (subtilisine BP N'; Carlsberg Res. Commun., vol. 41, n° 5, pages 238 à 241, 1976, I.B. SVENDSEN ).

Grâce au procédé suivant l'invention, on a pu préparer un hydrolysat riche en di et tripeptide se présentant sous la forme d'une masse pulvérulente de préférence blanche de saveur douce et goût agréable, contenant :

- 75 % en moles de di et tripeptides au moins;
- moins de 5 % en moles d'acides aminés:
- moins de 20 % en moles de peptides supérieurs à trois acides aminés, la longueur moyenne de chaîne étant égale à environ 6,2. De tels hydrolysats n'ont jamais pu être préparés par simple hydrolyse à partir de protéines animales et/ou végétales.

Des particularités et détails de l'invention relatifs aux divers substrats sont repris ci-dessous à titre d'exemples non limitatifs.

Les expérimentations suivantes sont réalisées à l'échelle du pilote dans un appareillage (de 20 à 300 litres de milieu réactionnel) préfigurant l'échelle industrielle. Les sources protéiques mises en oeuvre sont préparées comme indiqué ci-dessous.

## I. PLASMA SANGUIN ANIMAL.

Le sang se compose d'un liquide appelé plasma, dans lequel baignent des éléments figurés, constitués par les hématies ou globules rouges, les leucocytes ou globules blancs et les plaquettes sanguines. Après addition d'un anticoagulant, la centrifugation permet de recueillir d'une part le plasma (60 à 65% en vol.), d'autre part, les éléments figurés qui constituent le cruor (35 à 40% en vol.).

Le procédé de préparation d'une composition suivant l'invention à partir de sang d'abattage comprend les quatre étapes essentielles suivantes :

1. la mise en forme du plasma sanguin animal;
2. l'hydrolyse enzymatique;
3. l'extraction et purification d'une fraction riche en di et tripeptides: et
4. la stérilisation et le séchage de la fraction riche en di et tripeptides.

## 1.- LA MISE EN FORME DU PLASMA SANGUIN ANIMAL

Le sang total d'abattage est recueilli à l'abattoir sous anti-coagulant. On ajoute à cet effet au plus 4 g/l d'oxalate, de citrate ou de polyphosphate de potassium ou de sodium. Ce sang est ensuite soumis à une centrifugation.

Le plasma ainsi recueilli est soit utilisé immédiatement en l'état, soit immédiatement congelé ou séché en vue de sa conservation pour une utilisation différée.

Après remise en solution à une concentration d'environ 70 g/l (N x 6,25), le plasma congelé ou atomisé est ajusté à un pH inférieur à 6 et porté à une température supérieure à 65°C. Les conditions conduisant à une précipitation maximale des protéines plasmatiques sont retenues.

De préférence, on abaisse le pH du plasma animal entre 4,5 et 5,0 par un acide fort alimentaire (HCl, $H_2SO_4$ ou de préférence $H_3PO_4$ 4 à 6 Normal), tandis que la température de la solution de protéines est élevée à 70°C dans un intervalle de temps inférieur à 30 minutes environ, on maintient cette température pendant 30 minutes puis on l' abaisse rapidement (5 à 10 minutes) jusqu'à environ 20°C. Ensuite on récupère 95% en poids de l'azote protéique sous la forme d'un coagulat dont la teneur moyenne en protéines est de 160 à 220 g.kg$^{-1}$.

Lors de l'abaissement du pH du plasma animal, on emploie généralement de l'acide phosphorique car les ions phosphates ont la propriété de former des précipités insolubles avec les cations bivalents, notamment ceux apportés ultérieurement par les alcalis $Ca(OH)_2$ utilisés lors de l'hydrolyse enzymatique, par exemple. Ainsi il est possible d'abaisser de manière significative le taux de calcium dans le produit fini grâce à l'utilisation d'acide phosphorique lors de l'étape préliminaire de mise en forme du substrat.

Après traitement thermique, le plasma sanguin est soumis à une séparation liquide-solide.

La séparation liquide-solide est effectuée par microfiltration tangentielle, centrifugation ou filtration sur filtre-presse. Actuellement la filtration sur filtre-presse à cadres et plateaux donne les meilleurs résultats.

Ce traitement thermique suivi d'une séparation liquide-solide présente les avantages suivants :

- augmentation de l'accessibilité du substrat à l'enzyme;
- destruction de la majeure partie des inhibiteurs protéasiques:
- abaissement significatif de la flore mésophile contaminante;
- élimination des acides aminés libres et autres petites molécules de nature non protéique (lipides, sucres, urée..); et

-   diminution de la force ionique de la solution de protéines substrat après élimination des ions lors de la séparation liquide-solide par filtration ou centrifugation.

Le coagulat de protéines, partiellement dessalé, est utilisé immédiatement en réacteur enzymatique ou bien conservé au congélateur à -20 °C.

## 2.- HYDROLYSE ENZYMATIQUE.

### A. Choix de l'enzyme (protéase)

La réaction d'hydrolyse est optimale pour toute protéase permettant d'obtenir un taux de solubilisation des protéines plasmatiques supérieur à 90% en milieu acide (rapport protéines acido-solubles dans l'acide trichloracétique à 100 g.l$^{-1}$ sur protéines totales soumises à l'hydrolyse) ainsi qu'une concentration molaire en di et tripeptides représentant au moins 75% en moles de la fraction enrichie en di et tripeptides.

Les protéases les mieux adaptées sont de type protéases à sérine, alcalines, telles que celles issues de Bacillus licheniformis dont le composant majeur est la subtilisine de Carlsberg (EC 3. 4. 4. 16).

A titre d'exemple, les enzymes protéolytiques industrielles de type Alcalase 2, 4 1 (NOVO) ou Pescalase 560 (GIST BROCADES) peuvent être choisies.

Pour des valeurs de pH comprises entre 7 et 9, de préférence entre 7,0 et 7,5, la réaction d'hydrolyse du plasma bovin par l'Alcalase® (NOVO) ou par la Pescalase® (GIST BROCADES) est optimale. De plus, à ces valeurs de pH relativement voisines de la neutralité, les quantités d'alcalis nécessaires au maintien du pH durant la protéolyse, sont nettement moins importantes qu'à des pH alcalins (8 à 9). Cette observation n'est pas à négliger dans la mesure où l'élimination des ions, à la fin du procédé de fabrication, est difficile.

Lors de la réaction d'hydrolyse, les alcalis utilisés pour le maintien du pH peuvent être très divers (KOH, Ca(OH)$_2$, NaOH, etc). Afin de limiter la force ionique dans le produit résultant de l'hydrolyse, la chaux (Ca(OH)$_2$) sera généralement choisie comme alcali pour le contrôle et le maintien du pH.

Les températures de réaction les plus appropriées sont comprises entre 50 et 60 °C, de manière que la protéolyse s'effectue rapidement et que la dénaturation de l'enzyme soit minimale.

Cependant, le choix d'une température égale à 60 °C permet d'abaisser le temps de réaction (5 heures au lieu de 9 heures à 50 °C) et de limiter de façon plus significative la prolifération bactérienne durant l'hydrolyse.

La combinaison d'un pH neutre ou sensiblement neutre (7 à 7,5) et d'une température comprise entre 50 et 60 °C permet d'obtenir un taux de solubilisation des protéines plasmatiques en milieu acide supérieur à 90% et un taux de di et tripeptides d'au moins 75% pour des durées de réaction variant de 5 h à 9 h, ceci lorsque les protéases mises en oeuvre sont soit l'Alcalase® (NOVO), soit la Pescalase® (GIST BROCADES).

Le rapport pondéral est défini par l'équation suivante :

$$\frac{\text{Masse d'enzyme}}{\text{Masse de protéines substrat}} \times 100 = \frac{E}{S} \text{ en \%}$$

dans laquelle la masse d'enzyme est égale à la masse de préparation industrielle d'alcalase ou de pescalase introduite dans le milieu réactionnel. La masse de substrat est équivalente à la masse de protéines plasmatiques introduites dans le réacteur. Elle est mesurée par la méthode Kjeldhal (quantité d'azote élémentaire x 6,25).

Le rapport pondéral des concentrations respectives en enzyme et en protéines substrat doit être compris entre 1 et 4%.

En outre, il faut que la concentration en protéines substrat, dans le réacteur, soit la plus élevée possible, tout en permettant une bonne homogénéité du milieu.

A titre d'exemple, un rapport E/S de 2% et une concentration en substrat voisine de 90 g.l$^{-1}$ semblent optimum.

B. Destruction de l'enzyme.

Lorsque la réaction de protéolyse est considérée comme achevée, on procède à la destruction de l'enzyme en abaissant le pH entre 5,5 et 6,5 par addition d'acide fort, alimentaire (HCl, $H_2SO_4$, $H_3PO_4$...), de préférence $H_3PO_4$.

La combinaison d'un abaissement du pH du milieu avec une augmentation de la température est le meilleur compromis.

On augmente la température rapidement (en 10 à 20 minutes) jusqu'à une valeur comprise entre 70 et 80°C puis on maintient cette température pendant 5 à 45 minutes. Après ce traitement thermique, la température du milieu est rapidement abaissée jusqu'à une valeur comprise entre 15 et 20°C.

On abaisse de préférence le pH à 6,5 et on maintient pendant 20 minutes la température à 80°C. Les conditions opératoires particulières permettent une destruction complète de la protéase, sans qu'aucune trace d'activité ne soit détectable par les tests de référence, tout en évitant d'engendrer des dérivés toxiques tels que la lysinoalanine.

3.- PURIFICATION DE L'HYDROLYSAT ENRICHI EN DI- ET TRIPEPTIDES.

a. Clarification.

Après hydrolyse enzymatique du plasma animal, on soumet le milieu réactionnel à une clarification soit par centrifugation, soit par filtration (microfiltration tangentielle, filtration sur filtrepresse...), afin d'éliminer le résidu protéique insoluble, les lipides plasmatiques résiduels ainsi que des précipités minéraux tels que les phosphates de calcium.

On utilise par exemple un filtre-presse à cadres et à plateaux en présence d'adjuvant de filtration du type diatomite (Spendalite N) à une concentration de 30 à 50 g.kg$^{-1}$.

b. Ultrafiltration

L'hydrolysat clarifié par filtration est soumis à une étape d'ultrafiltration sur membranes de type organique (Polysulfone) conditionnées en modules de fibres creuses (Amicon), plans (Millipore) ou spirales (Millipore- Amicon) ou bien de type minéral (céramique) conditionnées en modules multicanaux (Tech Sep). On élimine par ultrafiltration, les protéines solubles partiellement hydrolysées ainsi que les peptides ayant une masse moléculaire supérieure à 10.000, avant de le soumettre à une opération de stérilisation et de séchage. La fraction peptidique recueillie dans l'ultrafiltrat constitue la composition riche en di et tripeptides. Le seuil de coupure des membranes doit être compris entre 1.000 et 10.000.

4.- FILTRATION STERILISANTE ET SECHAGE DE LA COMPOSITION RICHE EN DI ET TRIPEPTIDES (DTP)

Le mélange riche en di et tripeptides résultant de l'ultrafiltration est soumis, dans un délai aussi court que possible (n'excédant pas 24 h de conservation au réfrigérateur à + 4°C), à une filtration stérilisante ayant pour but d'éliminer tous les contaminants bactériens encore présents dans le mélange.

A cet effet, les systèmes de filtration de type Millipore, Sartorius,... peuvent être utilisés.

La filtrabilité du produit étant très bonne, le système de filtration à mettre en oeuvre est simple :
- un préfiltre de 2 à 0,65 microns;
- un filtre absolu 0,22 micron.

Le produit ainsi filtré est recueilli en récipient stérile avant d'être soumis à une déshydratation. L'élimination de l'eau peut être obtenue soit par lyophilisation, soit par atomisation couplée ou non à une préconcentration par évaporation sous vide.

La séparation et la quantification des peptides sur la base de leur nombre de résidus d'acides aminés constitutifs qui est indubitablement un problème difficile à résoudre, a été résolu par l'utilisation d'une technique récente de chromatographie d'échange de ligands haute performance (CELHP).

La chromatographie de perméation de gel est une technique spécifique récente. Elle ne sépare toutefois pas les petits peptides sur le seul critère de leur masse, parce qu'il y a recouvrement des zones d'élution des di- et tripeptides de masses différentes; ceci est lié notamment à l'existence d'interactions multiples peptides/gel. De plus, du fait des recouvrements des domaines de masse de familles de tailles différentes, la distribution des masses molaires ne permet pas de caractériser un mélange complexe de petits peptides sur base du critère de leurs tailles. Pour l'ensemble de ces raisons, la quantification des di et tripeptides n'est généralement pas réalisée correctement.

Par la mise au point d'une technique de chromatographie d'échange de ligand haute performance (CELHP) sur silice cuivrée couplée à la dégradation chimique d'Edman, un dosage direct des acides aminés et des dipeptides non basiques et un dosage indirect des tripeptides ont pu être effectués. La quantification des acides aminés des dipeptides non basiques et des peptides de tailles supérieures est réalisée par spectrométrie de fluorescence après réduction à l'état d'acides aminés.

L'hydrolysat obtenu par ce procédé suivant l'invention se présente sous la forme d'une masse pulvérulente blanche de saveur douce et goût agréable, ayant la composition suivante :
- 40 % en moles de di et 35 % en moles de tripeptides au moins;
- moins de 5 % en moles d'acides aminés; et
- moins de 20 % en moles d'oligo-peptides supérieurs à trois acides aminés, la longueur moyenne de chaîne étant égale à environ 6,2;
- osmolalité de 250 milliosmoles/litre à une concentration de 11,2 g d'azote libre par litre.

La matière sèche de la poudre obtenue en fin de traitement est de 94-96%.

Dans le paragraphe suivant, un procédé suivant l'invention a été appliqué à du lactosérum ainsi qu'à divers isolats protéiques.

## II. LACTOSERUM, ISOLAT DE POISSON, ISOLAT DE POIS, ISOLAT DE SOYA ET ISOLAT DE FROMENT.

Divers substrats ont été traités dans un procédé suivant l'invention. Ces substrats étaient entre autres :
- le lactosérum ou petit lait, c'est-à-dire un sous- produit de fromagerie obtenu lors de la séparation liquide-solide de la caséine insolubilisée par voie chimique ou biologique;
- un concentrat de protéines de poisson se présentant sous la forme d'une poudre de couleur beige clair, peu hygroscopique contenant 72 à 74 % en poids de protéines, 3 à 6 % de matières grasses, 12 à 15 % de matières minérales et 3 à 5% d'humidité résiduelle;
- un isolat de pois constitué d'extraits végétaux naturels fabriqués à partir de grains de pois sélectionnés et décortiqués;
- un isolat de froment se présentant sous la forme d'une poudre blanche ou beige au goût neutre contenant environ 86 % en poids de protéines; et
- un isolat de soja contenant plus de 90 % en poids de protéines.

Industriellement, les protéines de soja sont solubilisées préférentiellement puis séparées des coproduits insolubles. L'efficacité de la séparation liquide/solide entre la solution protéique et les co-produits insolubles conditionne le degré de purification de l'isolat préparé. Les protéines sont, dans une seconde étape, récupérées sélectivement par purification au point isoélectrique.

Le degré de purification des isolats protéiques de soja (et inversément les faibles teneurs en sels, lipides et fibres) permet d'éviter d'effectuer une étape supplémentaire de purification.

## 1. MISE EN FORME DES SUBSTRATS.

Les conditions de traitement thermique pour chacun des substrats étaient les suivantes :

Un concentré protéique de lactosérum, isolat de poisson, isolat de pois, isolat de soja et/ou isolat de froment a été acidifié à pH 4,6 par un acide fort alimentaire (HCl, $H_2SO_4$ ou de préférence $H_3PO_4$ 4 à 6 Normal) puis porté à 85-100°C en moins de 30 minutes environ et maintenu à cette températurte pendant une période de temps inférieure à 45 minutes à savoir 30 minutes. On a abaissé ensuite la température rapidement (5 à 10 minutes) jusqu'à environ 60°C. Un haut degré de purification du substrat et inversément une faible teneur en cendres permettaient de supprimer l'étape de séparation liquide-solide post-traitement thermique.

## 2.- HYDROLYSE ENZYMATIQUE.

Les conditions de l'hydrolyse enzymatique étaient :
- un pH neutre à alcalin, de 7 à 9;
- une température de 30 à 70°C, de préférence de 50 à 60°C;
- une durée de préférence variant entre 5 et 9 heures.

L'étape d'hydrolyse enzymatique était arrêtée par destruction de l'activité enzymatique, par exemple par abaissement du pH jusqu'à 5,5 à 6,5 par addition d'un acide fort alimentaire et chauffage à 75-80°C pendant 5 à 30 minutes.

Le procédé comprenait ensuite de manière similaire au procédé de traitement de plasma, une clarification, une ultrafiltration, une filtration stérilisante et un séchage.

Les compositions des substrats, les rendements azotés des étapes de clarification et d'ultrafiltration, les compositions des hydrolysats obtenus sont repris respectivement dans les tableaux 1 à 3.

**TABLEAU 1 :**    COMPOSITION DU SUBSTRAT AVANT TRAITEMENT THERMIQUE ET HYDROLYSE (% en poids)

| MATIERE PREMIERE | FOURNISSEUR | $H_2O$ % | Protéines % | Cendres % | Mat. Grasse % | Divers % |
|---|---|---|---|---|---|---|
| 1. Plasma sanguin | NORDFLEISCH(D) | 5-7 | 75 | 6 | | |
| 2. Lactosérum bovin PROTARMOR 90 | ARMOR PROTEINE (F) | 5 | 90 | 3 | 1,5 | 1* |
| 3. Isolat de poisson CORPESCA | SOPROPECHE (F) | 10 | 66 | 16 | 9 | |
| 4. Isolat de soya ARDEX SP 6 | ARKADY ADM (F) | 6 | 91.5 | 4.5 | 0.5 | |
| 5. Isolat de pois | COSUCRA (B) | 6 | 88 | 5 | 0.2 | |
| 6. Isolat de froment AMYPRO SWP | AMYLUM (B) | 5 | 86 | 5 | 8 | 1 |
| 7. Caséinate de Ca | ARMOR PROTEINE (F) | 6 | 88 | 4.5 | 1.5 | 0.2 |
| 8. Protéine blanc d'oeuf | IGRECA (F) | 8 | 78 | 6 | 0.5 | 0.7 |

* = % lactose

TABLEAU 2

| RENDEMENTS DES ETAPES DE CLARIFICATION ET D'ULTRAFILTRATION | | |
|---|---|---|
| Matière première | Rendement en azote après clarification | Rendement en azote après ultrafiltration |
| 1. Plasma sanguin | 80 % | 70 % |
| 2. Lactosérum bovin | 80 % | 70 % |
| 3. Isolat de poisson | 86 % | |
| 4. Isolat de soya | 73 % | 65 % |
| 5. Isolat de pois | 76 % | 69 % |
| 6. Isolat de froment | 76 % | |
| 7. Caséinate de Ca | 78 % | 68 % |
| 8. Protéines de blanc d'oeuf | 62 % | 58 % |

**TABLEAU 3 : COMPOSITION DES HYDROLYSATS EN ACIDES AMINES ET PEPTIDES**

| MATIERE PREMIERE | TENEUR RELATIVE EN MOLES - % | | | |
|---|---|---|---|---|
| | Acides aminés libres | dipeptides | tripeptides | oligopeptides plus grand que les tripeptides |
| 1. Plasma bovin | < 5 | > 40 | > 35 | < 20 |
| 2. Lactosérum bovin | < 5 | > 40 | 30-40 | 15-25 |
| 3. Isolat de poisson | < 7 | > 37 | 30-40 | 16-26 |
| 4. Isolat de soya | < 5 | > 43 | 30-35 | 17-22 |
| 5. Isolat de pois | < 4 | > 44 | 30-35 | 17-22 |
| 6. Isolat de froment | < 4 | > 48 | 30-35 | 13-18 |
| 7. Caséinate de Ca | < 4 | > 45 | 30-40 | 11-21 |
| 8. Protéines de blanc d'oeuf | < 3 | > 42 | 30-40 | 15-25 |

III. CASEINATE DE CALCIUM.

Les procédés actuellement utilisés pour extraire la caséine du lait sont basés sur l'insolubilisation de cette protéine par voie biologique ou chimique. On obtient ainsi une suspension de caséine dans le lactosérum facilement purifiable par séparation liquide-solide et lavage aqueux.

Cette étape de purification préliminaire de la caséine permet d'éviter une étape de séparation liquide-solide après le traitement thermique.

La caséine est une protéine peu soluble. La solubilisation de la caséine s'effectue industriellement à chaud et à pH basique. A l'issue de ce traitement dénaturant, les molécules de protéines sont réduites à l'état de monomère en solution aqueuse.

Le caséinate de calcium est issu de caséine fraîche résultant d'une fermentation lactique.

MISE EN FORME

Les conditions de mise en forme du substrat sont les suivantes :
- Chauffage à 90°C,
- Maintien du substrat à 90°C pendant 30 minutes d'une solution de caséinate de calcium à 77 g/l (N x 6,38) sans modification de pH (pH initial 6,8).

Dans cet exemple, on aurait également pu ajuster le pH à des valeurs voisines de la neutralité (pH 6 à 8).

HYDROLYSE ENZYMATIQUE

Ensuite, la température est abaissée jusqu'à 60°C et le pH de la solution aqueuse est ajusté à 7,5, au moyen d'une solution de chaux.

Le substrat est ensuite soumis à une hydrolyse enzymatique, une clarification, une ultrafiltration et une déshydratation dans les mêmes conditions que celles décrites pour les protéines plasmatiques.

Dans ces conditions opératoires, les débits de clarification et d'ultrafiltration sont habituels et témoignent d'une bonne digestion protéolytique (Tableau 2 - ligne 7).

Les résultats d'analyse de l'hydrolysat de caséinate de calcium sont groupés dans le tableau 3, à la ligne 7.

IV. PROTEINES DE L'OEUF.

Les protéines de blanc d'oeuf représentent un mélange d'une douzaine de protéines distinctes, de pH isoélectrique compris entre 4 et 11. De plus, certaines sont thermosensibles et gélifiantes tandis que d'autres sont thermorésistantes.

MISE EN FORME.

Afin d'éviter les inconvénients d'une gélification des protéines de blanc d'oeuf, le traitement thermique préalable comprend les étapes suivantes :

1) Ajustement à pH acide ou neutre (par exemple pH 6,00) par $H_3PO_4$ d'une solution de blanc d'oeuf à 85 g/kg.

2) Chauffage à 75°C du mélange sous agitation énergique et maintien pendant une période de temps inférieure à 45 minutes à savoir 30 minutes du mélange à la température susdite.

La température est ensuite abaissée jusqu'à 60°C et le pH est ajusté à 7,5 par une solution de chaux.

Ensuite, le procédé comprend les étapes décrites pour le processus des protéines plasmatiques à savoir une hydrolyse enzymatique la séparation et la purification de l'hydrolysat riche en di et tripeptides et le séchage.

La distribution moléculaire du mélange est identique à celle portant sur le produit d'hydrolyse des caséinates de calcium (tableau 3).

V. LACTOSERUM ADDITIONNE DE LACTOSE.

Les problèmes d'antigénicité rencontrés en nutrition spécialisée chez de jeunes enfants allergiques à des protéines lactées d'origine bovine sont réels.

Actuellement il est possible de s'affranchir de ce problème en soumettant les protéines du lait à un procédé d'hydrolyse conduisant à la disparition du caractère allergène. Cependant, lors de l'addition à l'hydrolysat de protéines d'un complément de lactose alimentaire représentant la fraction glucidique du nutriment il y a contamination de l'aliment par des protéines résiduelles (ceci malgré l'étape de purification du lactose par cristallisation). Celles-ci confèrent alors un caractère allergène à l'aliment.

L'hydrolyse enzymatique de protéines de lactosérum dans un milieu fortement enrichi en lactose selon le procédé suivant l'invention va permettre de produire un mélange hydrolysat peptidique-lactose directement utilisable en nutrition infantile.

Les conditions opératoires de la mise en forme de l'hydrolyse enzymatique et des traitements ultérieurs sont identiques à celles décrites pour le lactosérum et donnent des résultats semblables.

Par exemple, le taux de lactose ajouté au lactosérum peut atteindre 300 % en poids par rapport au poids de lactosérum.

## Revendications

1. Procédé pour préparer un hydrolysat enzymatique riche en di et tripeptides à partir d'un mélange de protéines à l'aide d'enzymes protéolytiques, caractérisé en ce qu'on met le mélange de protéines de départ en forme par traitement thermique dans un intervalle de température compris entre 70 et 100°C à pH 4,5 à 6 pour le plasma sanguin, a pH 4,0 à 5,0 pour le lactosérum, l'isolat de poisson, l'isolat de pois, l'isolat de soja et l'isolat de froment, à pH 6 à 8 pour le caséinate et à pH 6-7 pour les protéines de l'oeuf entre 30 et moins de 45 minutes et on soumet le mélange mis en forme à une hydrolyse enzymatique, à l'aide d'une seule enzyme additionnée en proportion de 1 à 4% en poids par rapport au substrat, de manière à obtenir un hydrolysat contenant 75% en moles de di et tripeptides avec un rendement d'hydrolyse d'au moins 70% en poids d'hydrolysat par rapport au mélange de protéines, on extrait l'hydrolysat par séparation liquide/solide, puis on en élimine, par ultrafiltration, des protéines solubles partiellement hydrolysées ainsi que les peptides ayant une masse moléculaire supérieure à 10000, de manière à réduire la teneur en acides aminés libres à moins de 5% en moles et la teneur en polypeptides supérieurs à 3 acides aminés à moins de 20% en moles, sans altérer le rendement net en di-et tripeptides du procédé.

2. Procédé selon la revendication 1, caractérisé en ce que la mise en forme de la source protéique de départ comprend un traitement thermique dans un intervalle de température compris entre 70 et 100°C à pH initial ou après ajustement à pH acide ou voisin de la neutralité.

3. Procédé selon l'une quelconque des revendications précédentes, caractérisé en ce que le substrat ou mélange de protéines mis en forme par traitement thermique en vue d'augmenter la vitesse de protéolyse, est hydrolysé à des valeurs de pH comprises entre 6,5 et 9, de préférence entre 7,0 et 7,5 à des températures comprises entre 30 et 70°C, de préférence entre 50 et 60°C pendant 5 à 9 heures.

4. Procédé selon l'une quelconque des revendications précédentes, caractérisé en ce que l'enzyme utilisé pour l'hydrolyse est une protéase utilisable en milieu alcalin.

5. Procédé selon la revendication 4, caractérisé en ce que la protéase est une protéase alcaline à serine.

6. Procédé selon l'une quelconque des revendications précédentes, caractérisé en ce qu'on procède à la destruction de l'enzyme, lorsque la réaction d'hydrolyse est considérée comme achevée, en abaissant le pH entre 5,5 et 6,5 par addition d'acide fort, alimentaire (HCl, $H_2SO_4$, $H_3PO_4$...) de préférence $H_3PO_4$, à un température comprise entre 70 et 80°C pendant 5 à 45 minutes.

7. Procédé selon l'une quelconque des revendications précédentes, caractérisé en ce qu'on filtre le mélange de protéines mis en forme par traitement thermique sur filtre presse à cadre et plateaux en vue d'éliminer les acides aminés libres, et autres molécules de bas poids moléculaire de nature non protéique (lipides, sucres, urée) et obtenir un mélange de di et tripeptides final ayant une faible teneur en acides aminés libres et une faible force ionique.

8. Procédé selon l'une quelconque des revendications précédentes, caractérisé en ce qu'on soumet du plasma sanguin, après ajustement à pH acide, à un traitement thermique dans un intervalle de température compris entre 70 et 100°C pendant une période de temps inférieure à 45 minutes, et ensuite à une séparation liquide-solide, par microfiltration tangentielle ou centrifugation, filtration sous pression ou dépression (de préférence par filtration sur filtre-presse à cadres et plateaux) de manière à obtenir un substrat solide pouvant être soumis à l'hydrolyse en présence d'enzymes protéolytiques après remise en suspension.

9. Procédé selon la revendication 8, caractérisé en ce que le plasma sanguin soumis au traitement thermique est préparé en ajoutant moins de 4 g/l d'anti-coagulant à du sang d'abattage et en soumettant ensuite ledit sang à une centrifugation de manière à obtenir un produit utilisé extemporanément ou apte à la conservation par congélation ou séchage par atomisation.

10. Procédé selon l'une quelconque des revendications 1 à 7, caractérisé en ce qu'on soumet une matière première constituée de protéines de lactosérum à un traitement thermique préalable après ajustement du pH à 4,0-5,0, en particulier à pH 4,6 à une température de 85 à 100°C pendant une période de temps inférieure à 45 minutes afin d'obtenir une suspension protéique que l'on soumet ensuite à hydrolyse en présence d'enzymes protéolytiques.

11. Procédé selon la revendication 10, caractérisé en ce qu'on ajoute du lactose alimentaire aux protéines de lactosérum avant le traitement thermique préalable.

12. Procédé selon l'une quelconque des revendications 1 à 7, caractérisé en ce qu'on soumet une matière première constituée de protéines choisies parmi les protéines de soya, pois, froment ou d'isolat de poisson à un traitement thermique après ajustement du pH à 4-5, en particulier 4,6 à une température de 70 à 100°C pendant une période de temps inférieure à 45 minutes afin d'obtenir une suspension protéique que l'on soumet ensuite à hydrolyse en présence d'enzymes protéolytiques.

13. Procédé selon l'une quelconque des revendications 1 à 7, caractérisé en ce qu'on soumet le caséinate à pH 6 à 8, de préférence à pH 6,8 à un traitement thermique à une température de 70 à 100°C pendant une période de temps inférieure à 45 minutes.

14. Procédé selon l'une quelconque des revendications 1 à 7, caractérisé en ce qu'on soumet les protéines de blanc d'oeuf ajustés à pH acide ou neutre à un traitement thermique à une température de 70 à 100°C pendant une période de temps inférieure à 45 minutes.

15. Procédé selon la revendication 1 ou 2, caractérisé en ce que le traitement thermique de la source protéique est effectué pendant une période de temps inférieure à 45° minutes.

16. Hydrolysat contenant des di et tripeptides obtenu par un procédé selon l'une quelconque des revendications précédentes, à partir de protéines animales et/ou végétales, caractérisé en ce qu'il se présente sous la forme d'une masse pulvérulente contenant :
   - 75 % en moles de di et tripeptides au moins;
   - moins de 5 % en moles d'acides aminés;
   - moins de 20 % en moles de peptides supérieurs à trois acides aminés, la longueur moyenne de chaîne étant égale à environ 6,2.

## Claims

1. Process for preparing a di- and tripeptide-rich enzymatic hydrolysate from a protein mixture, in the presence of proteolytic enzymes, characterized in that the starting protein mixture is conditioned by heat treatment with a temperature between 70 and 100°C at a pH comprised between 4.5 and 6 for blood plasma, at pH 4,0 to 5,0 for milk serum, fish isolate, pea isolate, soybean isolate and wheat isolate, at pH 6 to 8 for caseinate and at pH 6-7 for egg proteins, during 30 and less than 45 minutes and the conditioned mixture is subjected to enzymatic hydrolysis with the help of one sole enzyme, added in a rate of 1 to 4 weight-% in regard to the substrate, in such a manner that a hydrolyse yield of at least 70 weight-% is obtained in regard to the protein mixture, the hydrolysate is extracted by liquid/solid separation, and then the partially hydrolysed soluble proteins as well as the peptides with a molecular mass of more than 10.000 are removed therefrom by infiltration in order to decrease the amino-acids concentration to less than 5 mol.% and the concentration of polypeptides of more than three amino-acids to less than 20 mol.% without affecting the net di- and tripeptides yield of the method.

2. Process according to claim 1, characterized in that the conditioning of the starting protein source comprises a heat treatment in a temperature range between 70 and 100°C, at the initial pH or after adjusting to an acidic or close to neutral pH.

3. Process according to any one of the preceding claims, characterized in that the substrate or protein mixture conditioned by heat treatment in order to increase the rate of proteolysis, is hydrolysed at pH values of between 6.5 and 9, preferably between 7.0 and 7.5 at temperatures of between 30 and 70°C, preferably between 50 and 60°C, for 5 to 9 hours.

4. Process according to any one of the preceding claims, characterized in that the enzyme used for the hydrolysis is a protease which can be used in an alkaline medium.

5. Process according to claim 4, characterized in that the protease is a serine-containing alkaline protease.

6. Process according to any one of the preceding claims, characterized in that the destruction of the enzyme is carried out, when the hydrolysis reaction is considered to be complete, by reducing the pH to between 5.5 and 6.5 by the addition of a strong food acid (HCl, $H_2SO_4$, $H_3PO_4$ and the like) preferably $H_3PO_4$, at a temperature of between 70 and 80°C, for 5 to 45 minutes.

7. Process according to any one of the preceding claims, characterised in that the protein mixture conditioned by heat treatment is filtered on a plate and frame filter press in order to remove the free amino acids and other low molecular weight molecules of nonprotein nature (lipids, sugars and urea) and obtain a final di- and tripeptide mixture having a low free amino acid content and low ionic strength.

8. Process according to any one of the preceding claims, characterised in that blood plasma is subjected, after adjusting to acidic pH, to a heat treatment in a temperature range between 70 and 100°C for a period of less than 45 minutes, and then to liquid/solid separation by tangential microfiltration or by centrifugation, or by filtration under pressure or vacuum (preferably by plate and frame filter press filtration) in order to obtain a solid substrate which can be subjected to hydrolysis in the presence of proteolytic enzymes after resuspension.

9. Process according to claim 8, characterised in that the blood plasma subjected to heat treatment is prepared by adding less than 4 g/l of anticoagulant to the slaughtering blood and then by subjecting the said blood to centrifugation in order to obtain a product which is used immediately or which is suitable for preservation by freezing or drying by spray-drying.

10. Process according to claims 1 to 7, characterised in that a raw material consisting of milk serum proteins is subjected to a prior heat treatment after adjusting the pH to 4.0-5.0, in particular to pH 4.6, at a temperature of 85 to 100°C, for a period of less than 45 minutes, in order to obtain a protein suspension which is then subjected to hydrolysis in the presence of proteolytic enzymes.

11. Process according to claim 10, characterised in that food lactose is added to the milk serum proteins before the prior heat treatment.

12. Process according to any one of claims 1 to 7, characterised in that a raw material consisting of proteins chosen from soybean, pean wheat or fish isolate proteins is subjected to a heat treatment after adjusting the pH to 4-5, in particular 4.6, at a temperature of 70 to 100°C for a period of less than 45 minutes, in order to obtain a protein suspension which is then subjected to hydrolysis in the presence of proteolytic enzymes.

13. Process according to any one of claims 1 to 6, characterised in that caseinate is subjected, at pH 6 to 8, preferably at pH 6.8, to a heat treatment, at a temperature of 70 to 100°C for a period of less than 45 minutes.

14. Process according to any one of claims 1 to 6, characterised in that egg white proteins adjusted to pH 6.0 are subjected to a heat treatment at a temperature of 70 to 100°C for a period of less than 45 minutes.

15. Process according to claim 1 or 2, characterised in that the heat treatment of the protein source is carried out for a period of less than 45 minutes.

**16.** Hydrolysate containing di- and tripeptides obtained by a method according to any one of the preceding claims from animal and/or vegetable proteins, characterised in that it is provided in the form of a pulverulent mass, containing :
- not less than 75 mol % of di- and tripeptides;
- less than 5 mol % of amino acids;
- less than 20 mol % of peptides of more than three amino acids, the mean chain length being equal to about 6.2.

**Patentansprüche**

1. Verfahren zur Gewinnung von Enzymhydrolysat mit erhöhtem Gehalt an Di- und Tripeptiden aus einer Proteinmischung mit Hilfe von proteolytischen Enzymen, dadurch gekennzeichnet, daß die Proteinmischung zunächst während einer Zeit zwischen 30 und weniger als 45 Minuten bei Temperaturen zwischen 70° und 100° C bis zur Erreichung eines pH-Wertes von 4,5 bis 6 für Blutplasma, 4,0 bis 5,0 für Molke (Lactoserum) und Fisch-, Erbsen-, Soja- oder Weizen-Extrakt, von 6 bis 8 für Kasein und von 6 bis 7 für Ei-Proteine thermisch vorbehandelt und die so vorbereitete Mischung durch Zusatz eines einzigen Enzyms in einer Menge von 1 bis 4 Gew.-%, bezogen auf das Substrat, einer enzymatischen Hydrolyse unterworfen wird bis zur Erzielung eines einen Gehalt von 75 Mol-% an Di- und Tripeptiden und einen Hydrolysationsgrad von mindestens 70 Gew.-% an Hydrolysat, bezogen auf die Protein-Mischung, aufweisenden Hydrolysats, worauf die feste von der flüssigen Phase getrennt und anschließend durch Ultrafiltration die teilhydrolysierten löslichen Proteine sowie die Peptide mit einem Molekulargewicht über 10 000 abgetrennt werden bis auf einen Gehalt von unter 5 Mol-% an freien Amino-Säuren und von unter 20 Mol-% an Polypeptiden mit mehr als 3 Aminosäure-Resten, ohne Verminderung des Rein-Ertrages des Verfahrens an Di- und Tripeptiden.

2. Verfahren nach Anspruch 1, dadurch gekennzeichnet, daß die Vorbehandlung des Protein-Ausgangsmaterials durch Erwärmung auf eine Temperatur zwischen 70° und 100° C bis zum Ausgangs-pH-Wert und anschließende Einstellung des pH-Wertes auf einen im sauren oder neutralen Bereich liegenden Wertes erfolgt.

3. Verfahren nach einem der vorangehenden Ansprüche, dadurch gekennzeichnet, daß das zum Zwecke der Beschleunigung der Proteolyse thermisch vorbehandelte Substrat bzw. die vorbehandelte Proteinmischung bei einem pH-Wert zwischen 6,5 und 9, vorzugsweise zwischen 7,0 und 7,5 und einer Temperatur zwischen 30° und 70° C, vorzugsweise zwischen 50° und 60° C während einer Zeit von 5 bis 9 Stunden hydrolysiert wird.

4. Verfahren nach einem der vorangehenden Ansprüche, dadurch gekennzeichnet, daß als Enzym für die Hydrolyse eine in alkalischem Medium wirksame Protease Verwendung findet.

5. Verfahren nach Anspruch 4, dadurch gekennzeichnet, daß als Protease eine alkalische Serin-Protease Verwendung findet.

6. Verfahren nach einem der vorangehenden Ansprüche, dadurch gekennzeichnet, daß nach Beendigung der hydrolytischen Reaktion die Enzyme zerstört werden durch Erniedrigung des pH-Wertes auf einen Wert zwischen 5,5 und 6,5 durch Zusatz einer starken Speise-Säure (HCl, $H_2SO_4$, $H_3PO_4$, ...), vorzugsweise $H_3PO_4$, bei einer Temperatur zwischen 70° und 80° C während einer Zeit zwischen 5 und 45 Minuten.

7. Verfahren nach einem der vorangehenden Ansprüche, dadurch gekennzeichnet, daß die durch Erwärmung vorbehandelte Proteinmischung mittels einer Rahmen- oder Kammer-Filterpresse filtriert wird zur Beseitigung der freien Aminosäuren und anderer nicht-proteinischer Moleküle mit niedrigem Molekulargewicht (Lipide, Zucker, Harnstoff) und Erzielung einer abschließenden Mischung aus Di- und Tripeptiden mit geringem Gehalt an freien Aminosäuren und geringer Ionen-Stärke.

8. Verfahren nach einem der vorangehenden Ansprüche, dadurch gekennzeichnet, daß Blutplasma nach Einstellung des pH-Wertes einer Wärmebehandlung in einem Temperaturbereich zwischen 70° und 100° C während einer Zeit unter 45 Minuten unterworfen und anschließend die feste von der flüssigen Phase mittels eines Micro-Zentrifugalfilters, Druck- oder Vakuum-Filters (vorzugsweise einer Rahmen- oder Kammer-Filterpresse) getrennt werden zur Erzielung eines festen, nach Rückführung in Lösung für die Hydrolyse in Anwesenheit eines proteolytischen Enzyms geeigneten Substrats.

9. Verfahren nach Anspruch 8, dadurch gekennzeichnet, daß thermisch vorbehandeltes Blutplasma mit einem Gerinnungshemmer in einer Menge unter 4 g/l Feststoffgehalt versetzt und anschließend zentrifugiert wird zur Gewinnung eines unmittelbar einsetzbaren oder für die Konservierung durch Gefrier- oder Sprühtrocknung geeigneten Produktes.

10. Verfahren nach einem der Ansprüche 1 bis 7, dadurch gekennzeichnet, daß aus Molke gewonnenes Ausgangsmaterial nach Einstellung eines pH-Wertes von 4,0 - 5,0, vorzugsweise 4,6, bei einer Temperatur von 85° bis 100° C während einer Zeit unter 45 Minuten vorbehandelt wird zur Erzielung einer anschließend der Hydrolyse in Anwesenheit proteolytischer Enzyme unterworfenen Protein-Syspension.

11. Verfahren nach Anspruch 10, dadurch gekennzeichnet, daß Proteinen der Molke vor der thermischen Vorbehandlung Speise-Laktose zugefügt wird.

12. Verfahren nach einem der Ansprüche 1 bis 7, dadurch gekennzeichnet, daß aus Proteinen von Fisch-, Erbsen-, Soja- oder Weizen-Extrakten gewonnenes Ausgangsmaterial nach Einstellung eines pH-Wertes von 4,0 - 5,0, vorzugsweise 4,6, bei einer Temperatur von 70° bis 100° C während einer Zeit unter 45 Minuten thermisch vorbehandelt wird zur Erzielung einer anschließend der Hydrolyse in Anwesenheit proteolytischer Enzyme unterworfenen Protein-Syspension.

13. Verfahren nach einem der Ansprüche 1 bis 7, dadurch gekennzeichnet, daß Caseinate bei einem pH-Wert von 6 - 8, vorzugsweise 6,8 und einer Temperatur von 70° bis 100° C während einer Zeit unter 45 Minuten thermisch vorbehandelt werden.

14. Verfahren nach einem der vorangehenden Ansprüche, dadurch gekennzeichnet, daß auf einen sauren oder neutralen pH-Wert eingestellte Eiweiß-Proteine einer thermischen Behandlung bei einer Temperatur von 70° bis 100° C während einer Zeit unter 45 Minuten unterworfen werden.

15. Verfahren nach Anspruch 1 oder 2, dadurch gekennzeichnet, daß thermische Behandlung des proteinischen Ausgangsmaterials während einer Zeit unter 45 Minuten erfolgt.

16. Di- Tripeptide enthaltendes Hydrolysat, das mittels eines Verfahrens nach einem der vorangehenden Ansprüche aus tierischen und/oder pflanzlichen Proteinen gewonnen worden ist, dadurch gekennzeichnet, daß es in Form einer pulverigen Masse vorliegt und
   - mindestens 75 Mol-% an Di- und Tripeptiden
   - weniger als 5 Mol-% Aminosäuren
   - weniger als 20 Mol-% an Peptiden mit mehr als drei Aminosäure-Gruppen einer mittleren Kettenlänge von etwa 6,2
   enthält.